# EUROPEAN PATENT APPLICATION

(11) **EP 4 667 102 A2**
(43) Date of publication of application: **24.12.2025**
(21) Application number: 25183664.9
(22) Date of filing: 18.06.2025
(51) Int. Cl.: B01L 3/00

(54) **TEST DEVICE FOR TESTING ANALYTE IN LIQUID SAMPLE**

(30) Priority: 18.06.2024 CN 202410791510; 26.06.2024 US 202463664312 P; 22.08.2024 CN 202411159969; 05.09.2024 US 202463691020 P
(71) Applicant: Zhejiang Orient Gene Biotech Co., Ltd, Anji Huzhou Zhejiang 313300 (CN)
(72) Inventor: Fang, Jianqiu, Huzhou, Zhejiang, 313300 (CN); Lei, Siyu, Huzhou, Zhejiang, 313300 (CN); Zhang, Ying, Huzhou, Zhejiang, 313300 (CN); Shen, Lili, Huzhou, Zhejiang, 313300 (CN)
(74) Representative: Petraz, Gilberto Luigi

(57) **Abstract**

Provided in the present application is a test device, including a base layer. The base layer is provided with a slot for accommodating a testing element. The base layer further includes a structure for retaining a liquid sample. By means of the test device, an analyte in the liquid sample can be tested quickly, efficiently, and accurately, and an operator can use the test device more conveniently and freely without causing incorrect test results.

## Description

### CROSS-REFERENCE TO RELATED APPLICATIONS

The present application claims priority to Chinese Prior Application No. 2024107915109, filed on 18 June 2024; Chinese Prior Application No.2024111599693, filed on 05 September 2024; and US Provisional Application No. 63/691.020, filed on 05 September 2024; US Provisional Application No. 63/664.312, filed on 26 June 2024, and the abstract, drawings, claims and specification of which are incorporated herein by reference in their entirety as part of the present application.

### BACKGROUND OF THE INVENTION

### Field of the Invention

The present application relates to a device and method for testing an analyte in a liquid sample.

### Description of the Related Art

The following background is used to help readers understand the present application and should not be considered as prior art.

In our society, abuse of illegal drugs has become a recognized and deteriorating social problem. In 2003, a survey by the US Department of Health and Human Services found that about 19.5 million Americans or 8.2% of people over the age of 12 were taking illegal drugs. "Recent use of illegal drugs" refers to the use of an illegal drug within one month before the survey by the US Department of Health and Human Services. Marijuana was found to be the most commonly used illegal drug, accounting for 6.2% (14.6 million). It is estimated that 2.3 million people (1.0%) are currently taking cocaine, 604,000 people are taking crack, 1 million people are using hallucinogens, and 119,000 people are taking heroin.

In order to combat abuse of drugs and monitor this social problem, drug testing has become a standard testing procedure in various industries such as employment, education, sports, and law enforcement. In order to promote this effort, the drug testing industry has been formed. The industry offers a wide range of drug test products. A urine sample collection cup that can analyze samples is a classic test product. These devices may be complicated, difficult, or dirty for users, or may cause the problem of sample adulteration in order to conceal the recent use of illegal drugs. In addition, urine samples cannot be collected on some occasions, such as at the roadside or in public places.

As many other sample collection and test devices are inefficient in extracting samples from the collection devices, they always have many problems, such as environmental pollution due to sample leakage, test results affected due to too few or too many samples collected, or complicated detection due to many operation steps. Many of these devices are also very complicated in their design and manufacture, for which quite expensive materials need to be used. Therefore, better methods and devices are needed to collect and test samples.

### BRIEF SUMMARY OF THE INVENTION

In order to solve the problems in the prior art, provided in the present application is a test device for testing an analyte in a liquid sample and a method for testing an analyte in a liquid sample by using the test device. The use of the device and the test method can avoid many problems, and an operation method with superior performance and more reliable test results can be provided. In particular, the test results can be obtained quickly, and the operation is simpler.

In one aspect of the present application, the present application provides a test device, including a base layer, provided with a slot for accommodating a testing element, wherein the test device further includes a liquid retaining structure for retaining a liquid sample to be supplied to the testing element for absorption. The liquid retaining structure functions in such a way that when one end with the liquid retaining structure is inserted into the liquid sample, a part of the liquid is in contact with a test strip inside the slot, and the other part of the liquid enters the liquid retaining structure; once the test device is withdrawn from the liquid sample, the liquid within the liquid retaining structure can continue to be supplied to the test strip for absorption so as to complete a test. In this way, the test device can be rapidly inserted into the liquid and simultaneously rapidly withdrawn, preventing test failure caused by failing to fully wet the test strip due to a reduction in the liquid amount due to fast operation.

In some embodiments, the testing element is arranged in the slot, and a part of a liquid sample application area of the testing element is located in the liquid retaining structure. In some embodiments, the test device further includes a covering layer, and the covering layer covers a part of the slot located in the base layer to form a partial channel with one sealed end and one open end. In some embodiments, the other part of the slot not covered by the covering layer forms an open slot or extends outward from an opening of the channel. The other part of the slot not covered by the covering layer serves as a retaining structure for the liquid sample. When the open end of the channel is inserted into the liquid sample, the open slot (the other part of the slot not covered by the covering layer) can absorb and retain a part of the liquid for the test strip to absorb so as to complete the test. At this time, if the open slot also has a test strip, then the test strip also simultaneously is in contact with the liquid and absorbs a part of the liquid. After the device is rapidly withdrawn from the liquid sample, a part of the liquid is still retained in the open slot and can continue to be supplied to the test strip for absorption after the device is rapidly withdrawn from the liquid sample, so as to complete the test. Here, the "absorption" by the open slot means that the liquid sample is absorbed by the open slot and retained due to surface tension.

If the device is rapidly inserted into and rapidly withdrawn from the liquid sample, the liquid absorbed merely by contact with the test strip may not guarantee to wet the entire test strip, or the amount of liquid may be insufficient to dissolve a label pad and flow through a testing area to wet it. At this point, the liquid retaining structure can continue to supply the liquid to the test strip for absorption, ensuring the completion of the entire test. In this way, through rapid contact with the sample, a sufficient liquid sample can be obtained to complete the test.

In some embodiments, the other part of the slot not covered by the covering layer exists as an opening, which exposes the part of the sample application area of the testing element. In some embodiments, the end of the sample application area aligns with the end of the open slot, or, the entire slot has a length the same as the length of the testing element, while the part of the slot is covered by the covering layer to form a testing channel with one sealed end and one open end.

In some embodiments, to enable the open slot to retain the liquid, the width and depth of the slot can be configured. The primary purpose of this configuration is that after the open slot is inserted into the liquid sample and then withdrawn from the liquid sample (e.g., withdrawn within 1 to 3 seconds), a part of the liquid sample remains within the open slot to be supplied to the testing element for absorption. In some embodiments, the slot may be a slot with a rough surface. In some embodiments, since the part of the sample application area of the testing element is made of a filter paper or glass fiber material that may inherently serve as a rough surface. These rough materials, together with the open slot, form a space capable of retaining or holding a part of the liquid sample. The liquid is retained within the slot by utilizing liquid surface tension to be supplied to the test strip for continued absorption so as to complete the test.

In some embodiments, when the liquid sample is located in the open slot, the liquid does not flow out or is separated from the slot due to the liquid surface tension within the slot. In some embodiments, to enable the liquid to possess surface tension and prevent separation, specific limitations are imposed on the depth or width of the slot. When the sample is urine, the width of the open slot may be 1-6 mm, 2-5 mm, or 2, 3, 4, 5, or 6 mm, and the depth may also be 2-5 mm, for example, the depth is 1, 2, 3, 4, or 5 mm, at such size, the liquid remains within the open slot due to surface tension, after the open slot is inserted into the liquid and then withdrawn, a part of the liquid sample is retained within the open slot to be supplied to the test strip for continued absorption, the volume of the sample retained is approximately 1-50 microliters or 2-100 microliters. Such a liquid volume is sufficient for a test strip to absorb so as to complete the test. The complete the test means that at the liquid volume, the liquid can wet a label area and a testing area of the test strip, or can flow from the sample application area to the label area depending on capillary action to dissolve a label substance, and then flow to the testing area to wet the testing area.

In some embodiments, the part of the sample application area of the testing element is located in the open slot. In some embodiments, the base layer includes a back surface and a front surface, the slot is located in the back surface or the front surface of the base layer, and the covering layer covers the front surface or the back surface of the base layer. In some embodiments, the base layer is transparent, the front surface of the testing element covers a bottom of the slot, and the back surface of the testing element is covered and bonded by the covering layer.

In some embodiments, the liquid retaining structure includes a part of the open slot, and a width of the open slot is greater than a width of a sample application area of the test strip, such that a gap for retaining the liquid sample is formed through the slot and the sample application area.

In some embodiments, the slot includes two side walls arranged oppositely and a bottom formed by a bottom plate, the part of the sample application area is located on the bottom plate, and the two side walls have a width greater than a width of the sample application area to form some gaps, such that the gaps are capable of retaining the part of the liquid therein through surface tension to be supplied to the sample application area for continued absorption. Of course, the height of the two side walls may also be greater than the thickness of the sample application area of the testing element, thereby reserving space to retain the liquid sample.

In some embodiments, the liquid retaining structure includes a water absorbing and retaining material, wherein the water absorbing and retaining material covers the sample application area and is configured to absorb and retain the liquid sample, and the liquid sample is absorbed by the sample application area. In some embodiments, the absorbent material wraps a part of the sample application area, such that when the absorbent material of the sample application area is in contact with the liquid, the absorbent material absorbs the liquid sample to be supplied to the sample application area for absorption. In some embodiments, a volume of the liquid sample absorbed by the absorbent material is greater than or far greater than a volume required for wetting the entire test strip. In some embodiments, the volume of the liquid absorbed by the absorbent material is 1-100 times the volume required for wetting the test strip. In some embodiments, the liquid retaining structure includes an absorbent material, wherein the absorbent material covers the sample application area of each testing element and is configured to absorb and retain the liquid sample, and the liquid sample is absorbed by the sample application area. In some embodiments, the water absorbing and retaining material is located in the open slot. In some embodiments, the volume of liquid absorbed per unit volume of the water absorbing and retaining material is greater than or far greater than the amount of water per unit volume of the sample application area. In this way, only after the water absorbing and retaining material absorbs the liquid can the liquid be supplied to the sample application area for continued absorption. After all, the water absorbing and retaining material is essentially fully wetted after absorbing the liquid. The sample application area of the testing element may also absorb the liquid sample upon contact with the liquid sample. However, the label area, the testing area, or an absorption area are dry and also possess capillary force, such that the liquid in the water absorbing and retaining material can be allowed to flow onto the test strip for absorption by the label area, the testing area, or the absorption area to be wetted so as to complete the test. In some embodiments, when the sample application area is covered by the absorbent material in any of the above ways, the open clamping slot has two areas. In one area, the depth of the clamping slot may be the same as that of the sample application area and the absorbent material, while in the other area, the depth of the clamping slot is greater than the height of the sample application area.

In some embodiments, providing a water retaining structure near the opening of the channel or at the bottom of the slot can also have the function of retaining liquid. For example, the water retaining structure is a thread, a recession, a cavity, a hole, a slot, etc. Once these structures are in contact with the liquid sample, the liquid sample also relies on the liquid surface tension to enter these water retaining structures to be temporarily retained. After withdrawal from the liquid sample, the liquid sample within these water retaining structures will be absorbed by the test strip. In some embodiments, the water retaining structure is located at the bottom of the slot near the opening of the channel. In some embodiments, the covering layer covers the slot in the back of the base layer, and the back of the test strip is bonded by the covering layer. In some embodiments, the water retaining structure is in fluid communication with the back of the test strip. In some embodiments, the fluid communication means that the liquid in the water retaining structure is in contact with the absorbent material on the front of the test strip, and the absorbent material can absorb the liquid sample in the water retaining structure depending on the capillary force. In some embodiments, a liquid outlet of the water retaining structure or a water retaining cavity faces the front of the test strip, for example, facing the sample application area of the testing element. A distance, e.g., 2-3 mm, exists between the sample application area and the water retaining structure at the bottom of the slot. When the water retaining structure is inserted into the liquid sample, the liquid sample enters the water retaining structure through the distance between the sample application area and the bottom of the slot and is retained. After the test device is rapidly withdrawn from the liquid sample, the sample application area can allow the liquid in the water retaining structure to flow onto the testing element depending on the capillary force, so as to complete the test by the testing element.

In some embodiments, the liquid retaining structure includes a flow channel with two open ends, wherein a part of the channel includes the sample application area. In some embodiments, one open end of the flow channel is for the liquid sample to flow in, the other end is for venting gas, and the flow channel has a liquid retaining structure that allows the liquid to be within the channel. In some embodiments, the liquid retaining structure is a gap including a space limited by the sample application area and the flow channel. In some embodiments, the flow channel traverses the base layer and simultaneously traverses the testing channel, wherein the testing channel is covered by the covering layer to form the testing channel with one sealed end and one open end. In some embodiments, when the liquid passes through the gap, due to the liquid surface tension, the liquid cannot flow through the gap, such that the liquid is retained by the gap to continue to be supplied to the sample application area for absorption so as to complete the test reaction of the testing element. In some embodiments, the flow channel itself can serve as a liquid retaining structure. When the end with the flow channel is inserted into the liquid sample, the open end of the channel is for the liquid to flow in, and the other end can vent gas to allow the channel to fill with the liquid sample. Since the diameter of the channel is not very large, for example, 2-5 mm, once the device is withdrawn from the liquid sample, a part of the liquid sample is still retained within the flow channel, and the part of the liquid sample retained within the channel can continue to be supplied to the testing element for absorption.

In some embodiments, the flow channel is formed by the slot and the covering layer covering the slot, and open ends of the flow channel are arranged on the two sides of the base layer. In some embodiments, the flow channel includes a main flow channel, to which one or more subsidiary flow channels are connected in parallel, these multiple subsidiary flow channels are in fluid communication with the main flow channel. In some embodiments, a part of a sample application area is arranged in each subsidiary flow channel. In some embodiments, the main flow channel has a liquid inlet for the liquid to flow in, and the inflowing liquid flows into each subsidiary flow channel respectively, such that a part of the liquid is retained in the subsidiary flow channels to continue to be supplied to the test strip for liquid absorption. In some embodiments, one end of each subsidiary flow channel is connected to the main flow channel, and the other end is connected to the external atmosphere; when the liquid from the main flow channel flows into the subsidiary flow channels, air in the subsidiary flow channel can be vented into the atmosphere. In some embodiments, each subsidiary flow channel includes the liquid retaining structure. In some embodiments, the subsidiary flow channel itself is a liquid retaining structure; when the subsidiary flow channel is inserted into a sample, a liquid is retained by the subsidiary flow channel; after the subsidiary flow channel is withdrawn from the liquid, the liquid retained by the subsidiary flow channel continues to be supplied to the sample application area of the testing element for absorption so as to complete the test.

In some embodiments, the flow channel is formed by bonding two films, and the sample application area is located between the bonded films. In some embodiments, one end of the liquid retaining structure is located upstream of the label area of the testing element. In some embodiments, the test device further includes a structure for reducing, limiting, or eliminating capillary flow, and the structure prevents the liquid from flowing through a capillary gap formed between the test strip and the side walls of the slot. In some embodiments, the structure for reducing capillary flow is located on the side walls of the slot.

The present application further provides a method for testing an analyte in a liquid sample, including: providing the test device of any one of the aforementioned solutions; allowing a liquid retaining structure of the test device to enter the liquid sample; allowing the liquid sample to be absorbed or retained in the liquid retaining structure; withdrawing the test device; and reading test results on a testing area of a testing element. In some embodiments, the test results on the testing area are read to determine the presence or absence or quantity of the analyte in the sample. In some embodiments, the entire test device is soaked in the liquid sample for a period of time, and then withdrawn, and the test results on the testing element are read.

In some embodiments, the base layer is a rigid base layer, and the covering layer is a flexible or rigid covering layer. In some embodiments, the base layer has a specified thickness, and a thickness of the covering layer is less than the thickness of the base layer. In some embodiments, one end of the liquid retaining structure is located upstream of a label area of the testing element. In some embodiments, the test device further includes a structure for reducing, limiting, or eliminating capillary flow, and the structure prevents the liquid from flowing through a capillary gap formed between the test strip and the side walls of the slot. In some embodiments, the structure for reducing capillary flow is located on the side walls of the slot. In some embodiments, the structure for reducing, limiting, or eliminating capillary flow is a protruding tenon structure, such that a specified distance is kept between the test strip and the side walls of the slot, thereby reducing, limiting, and eliminating the capillary gap formed between the test strip and the side walls of the slot. In some embodiments, the structure for reducing, limiting, or eliminating capillary flow is located upstream of a label area of the testing element, downstream of a testing area, or upstream of a sample chamber. In some embodiments, the structure for reducing capillary flow is located upstream of the label area. In some embodiments, the sample chamber includes a support structure for supporting a liquid sample application area of the testing element, such that each area of a test strip is located on a same plane. In some embodiments, the slot further includes a venting structure therein, and when the liquid sample enters the slot, a part of gas in the slot is vented from the slot through the structure. In some embodiments, the venting structure is located on the bottom of the slot. In some embodiments, the venting structure is a slot structure. In some embodiments, one end of the venting structure is in communication with the slot while the other end thereof is in communication with external atmosphere. In some embodiments, one end of the venting structure is in communication with the slot while the other end thereof is in communication with an opening.

The present application provides a method for testing an analyte in a liquid sample, including: providing the aforementioned test device; allowing a liquid retaining structure of the test device to enter the liquid sample; allowing the liquid sample to be absorbed or retained in the liquid retaining structure; withdrawing the test device; and reading test results on a testing area of a testing element. In some embodiments, the test results on the testing area are read to determine the presence or absence or quantity of the analyte in the sample. A method for testing an analyte in a liquid sample is provided, and the method includes: providing the test device according to one of claims 1-25; soaking the entire test device in the liquid sample for a period of time; withdrawing the test device; and reading test results on a testing element.

### BRIEF DESCRIPTION OF THE DRAWINGS

Fig. 1 shows a schematic three-dimensional structural view of a test device according to a specific embodiment of the present application.
Fig. 2 shows a schematic partial structural enlarged view of a base layer of a device shown in Fig. 1.
Fig. 3 shows a schematic exploded view of a test device according to another specific embodiment of the present application.
Fig. 4 shows a schematic three-dimensional structural view of assembly of a test device according to a specific embodiment of the present application.
Fig. 5 shows a top view of a testing element according to a specific embodiment of the present application.
Fig. 6 shows a schematic exploded structural view of a test device according to a specific embodiment of the present application.
Fig. 7 shows a schematic exploded structural view of a test device according to a specific embodiment of the present application.
Fig. 8 shows a bottom view of a test device according to a specific embodiment of the present application.
Fig. 9 shows a schematic partially enlarged view of a base layer structure of a test device according to a specific embodiment of the present application.
Fig. 10 shows a schematic exploded structural view of a test device according to a specific embodiment of the present application.
Fig. 11 shows a schematic exploded structural view of a test device and a lid body according to a specific embodiment of the present application.
Fig. 12 shows a schematic structural view of a base layer of a test device according to a specific embodiment of the present application.
Fig. 13 shows a schematic exploded structural view of a test device according to a specific embodiment of the present application.
Fig. 14 shows a schematic exploded structural view of a test device according to a specific embodiment of the present application (with a base layer and a lid body disassembled).
Fig. 15 shows a schematic structural view of a base layer of a test device according to a specific embodiment of the present application.
Fig. 16 shows a schematic exploded structural view of a test device according to a specific embodiment of the present application.
Fig. 17 shows a schematic exploded structural view of a test device according to a specific embodiment of the present application (with a base layer and a lid body disassembled).
Fig. 18 shows a schematic structural diagram of a base layer of a test device according to a specific embodiment of the present application.
Fig. 19 shows a schematic partially enlarged view of a test device according to a specific embodiment of the present application.
Fig. 20 shows a schematic principle structural view of a test device according to a specific embodiment of the present application.
Fig. 21 shows a schematic principle structural view of a test device according to a specific embodiment of the present application.

### DETAILED DESCRIPTION OF THE INVENTION

The structures involved in the present application or the technical terms used are further explained below. These descriptions only illustrate how embodiments of the present application are achieved by way of example, and do not constitute any limitation on the present application.

### Testing

Testing means to assay or detect the presence or absence of a substance or material, including but not limited to, a chemical substance, an organic compound, an inorganic compound, a metabolite, a drug or a drug metabolite, an organic tissue or a metabolite of an organic tissue, a nucleic acid, a protein, or a polymer. In addition, testing means testing the quantity of a substance or material. Further, assay also means immunoassay, chemical assay, enzyme assay, and the like.

### Downstream and Upstream

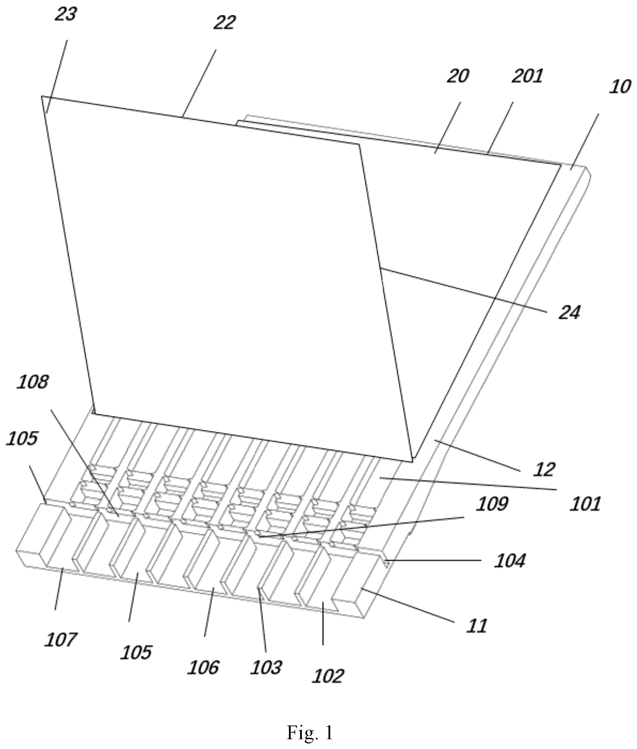

Downstream or upstream is divided according to the flow direction of a liquid. Generally, a liquid or fluid flows to a downstream area from an upstream area. The downstream area receives a liquid from the upstream area. A liquid may also flow to a downstream area along an upstream area. Here, downstream or upstream is generally divided according to the flow direction of a liquid, for example, on some materials where capillary force is utilized to facilitate the flow of a liquid, a liquid may overcome gravity to flow towards an opposite direction to the gravity; in this case, downstream or upstream is divided according to the flow direction of a liquid. For example, as shown in Fig. 1, a testing element 60 mentioned in the present application includes a sample application area 601, a label area 602, a testing area 603, and an absorption area 604. Generally, a liquid flows sequentially from the sample application area upstream to the label area downstream, then from the label area to the testing area downstream, and finally to the absorption area. In such a way, a liquid flows from upstream to downstream. The flow dynamic of a liquid depends on capillary action.

### Gas Communication or Liquid Communication

Gas communication or liquid communication means that a liquid or a gas can flow from one place to another. In the flow process, the liquid or the gas may pass through some physical structures that play a guiding role. The "passing through some physical structures" here generally means that the liquid passes through the surface of these physical structures or their inner space and flows to another place passively or actively, where passivity is generally caused by outer forces, such as flow under the capillary action and the action of air pressure. The flow here may also be a flow due to the self-action (gravity or pressure) of a liquid or gas, or a passive flow. The fluid under the action of air pressure may be a forward flow or a reverse flow; or a fluid is caused to flow from one position to another under the action of air pressure. The communication here does not mean that a liquid or a gas is necessarily present, but indicates a connection relationship or state between two objects in some cases. If a liquid is present, it can flow from one object to another. Here, it means a state in which two objects are connected. On the contrary, if the state of liquid communication or gas communication is not present between two objects, and if a liquid exists in or on one object but cannot flow into or onto another object, such a state is a non-communication, non-liquid communication, or non-gas communication state.

### Detachable Combination

A detachable combination means that two components are connected in several different states or positional relationships. For example, with two components being physical components, they can be separated at the beginning and connected or combined in an appropriate first case, and can be separated in an appropriate second case. Physically, such separation is spatial separation without contact. Alternatively, the two components are combined at the beginning, and can be physically spatially separated from each other when appropriate. Alternatively, two objects are separated at the beginning, combined to achieve a specified function if necessary, then separated, or later combined again for a purpose. In short, combination or separation of two components or two objects can be easily made and repeated many times. Of course, the combination or separation may also be single-use. In addition, such a combination may be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves are detachable or can be indirectly combined by other objects.

### Testing Element

Various testing elements can be combined for use in the present application. A testing element includes a test strip. The test strip can have various forms, for example, immunoassay or chemical test. The test strip is used for testing an analyte in a sample, such as drugs or related metabolites indicating physical conditions. In some forms, the test strip is an absorbent material with a liquid sample addition (application) area, a reagent area, and a test result area. A sample is added to the sample application area and flows to the reagent area under the capillary action. In the reagent area, the sample is dissolved in a reagent and mixed with the reagent for testing an analyte (if present in the sample). Of course, the reagent area and the sample application area may also be the same area, and some reagents for treating liquid samples are treated in the sample application area in advance. At this point, the sample with the reagent continues to flow to the test result area. Additional reagents are immobilized in the test result area. These reagents immobilized in the test result area react and bind with an analyte (if present) or with a first reagent in the reagent area. In a non-competitive test form, if an analyte is present in a sample, a signal will be generated; if an analyte is not present, no signal will be generated. In a competitive test form, if an analyte is not present in a sample, a signal will be generated; if the analyte is present, no signal will be generated. The present application is suitable for testing elements in various analysis forms.

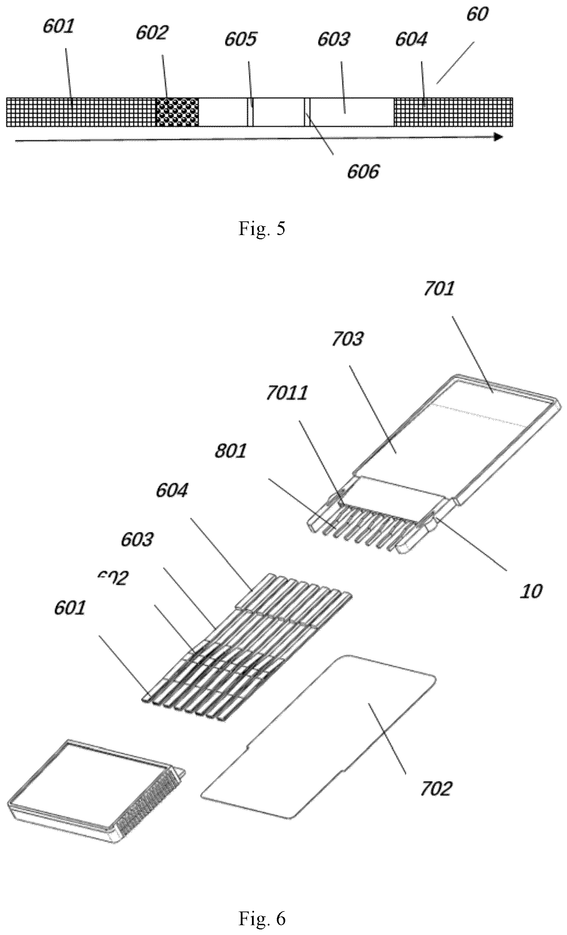

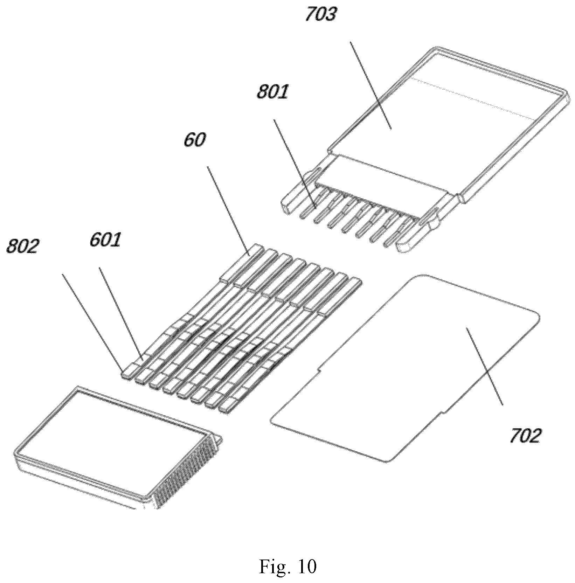

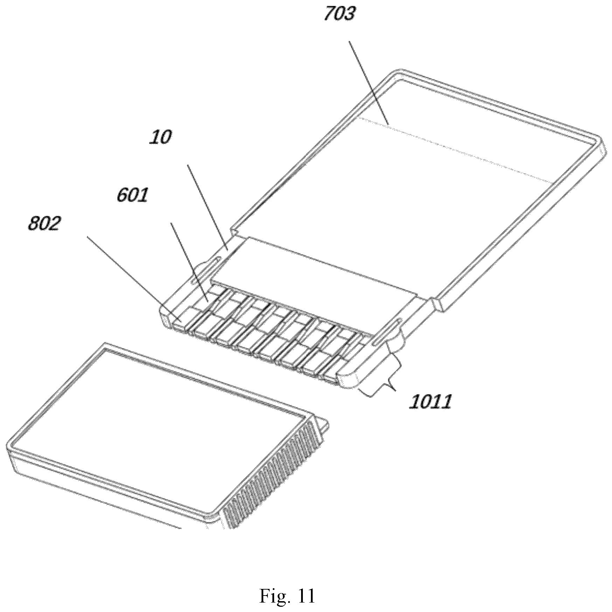

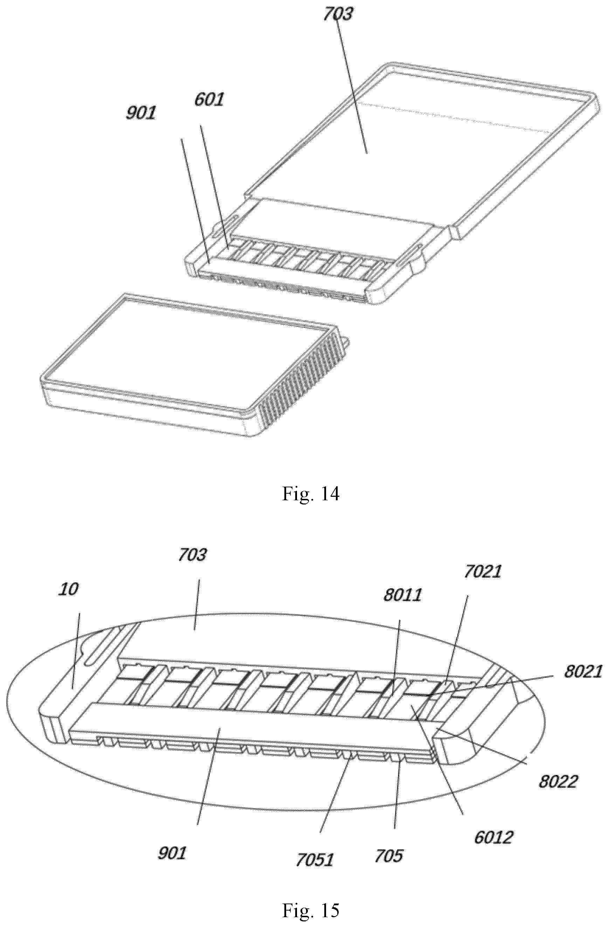

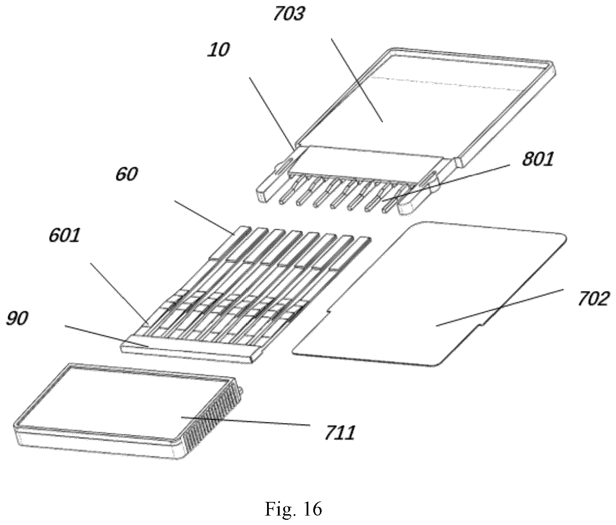

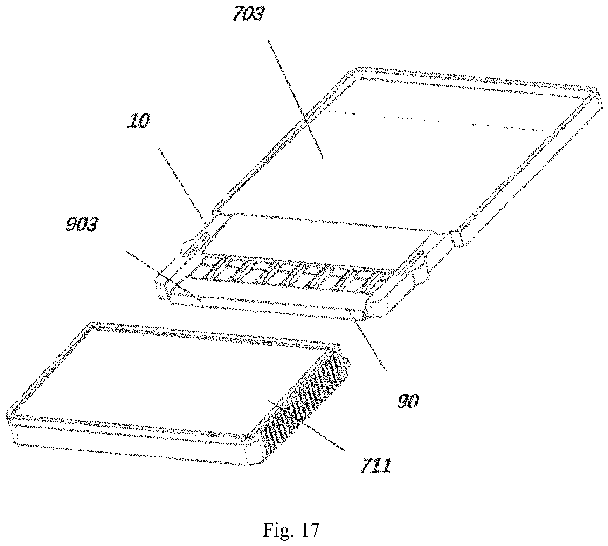

For example, as shown in Fig. 5, Fig. 6, and Figs. 10-21, when a testing element 60 is a test strip, it can be made of an absorbent or non-absorbent material. One test strip can be made of multiple materials for liquid transfer. One material of the test strip can be superimposed on another material thereof, for example, a filter paper is superimposed on nitrocellulose. Alternatively, an area including at least one material in the test strip is located behind another area including at least one different material. In this case, a liquid flows between the areas, and they can be superimposed on each other or not. The material on the test strip can be immobilized on a support, such as a plastic lining, or a hard surface to enhance the retention capability of the test strip.

In some embodiments where an analyte is tested by a signal generation system (for example, at least one enzyme reacts specifically with an analyte), at least one signal-generating substance can be adsorbed to an analyte testing area of the test strip, just as it is specifically adsorbed to the material of the test strip as described above. In addition, a signal-generating substance present in a sample application area 601, a label area 602, an analyte testing area 605, and a result control area 606 of the test strip, or throughout the entire test strip can be pretreated on one or more materials of the test strip in advance. This can be achieved by adding a signal-generating substance solution to the surface of the sample application area or soaking one or more materials of the test strip in the signal-generating substance solution. After the test strip is added with the signal-generating substance solution or soaked in this solution, the test strip is dried. In addition, through the above method, a signal-generating substance present in a sample application area 601, a reagent area, and an analyte testing area of the test strip, or throughout the entire test strip can be pretreated on one or more materials of the test strip in advance. In addition, a signal substance present in a sample application area, a reagent area, or a testing area of the test strip that serves as a label reagent can be added to one or more surfaces of the material of the test strip.

The areas of a test strip can be arranged as follows: a complete and necessary test strip may include a sample application area 601 and a testing area 603. Generally, a liquid is first in contact with the sample application area 601 and then flows to the testing area 603 based on capillary action. Of course, the test strip may also include the following areas as required: a sample addition area or sample application area 603, at least one label area 602, and at least one testing area 603, wherein the testing area includes a test result area 605, or further includes at least one control area 606, or may include at least one adulteration testing area and a liquid absorption area 604. If the testing area includes one control area, preferably, the control area is located behind an analyte testing area in the test result area. All these areas or combinations thereof can be provided on a single test strip including one material. In addition, these areas are made of different materials and connected together in the direction of liquid transfer. For example, a liquid may be directly or indirectly transferred through different areas. In this embodiment, different areas may be connected end to end in the direction of liquid transfer or superimposed with each other in the direction of liquid transfer, or connected by other materials, such as connecting medium materials (preferably absorbent materials such as filter paper, glass fiber, or nitrocellulose). In use, the connecting materials enable materials for connecting ends of areas, materials for connecting ends of areas without liquid flow, or materials for superimposing areas (for example, such superimposition is not limited to superimposition from the beginning of the areas to the ends of the areas) without liquid flow to form the liquid flow.

If the test strip includes an adulteration testing area, the adulteration testing area can be placed in front of or behind the test result area. When a result determination area includes a control area, the adulteration testing area is preferably placed in front of the control area, and this may not be the case. According to an embodiment of the present application, the test strip is a control test strip for adulteration analysis, judgment and/or control, and the adulteration testing area can be located in front of or behind a control area, preferably in front of a control area.

In a specific embodiment of the present application, any testing element or test strip can be located in a base layer slot or slot 101, or in a channel formed by a slot in a base layer covered by a covering element. The arrangement way of the test strip in the test device of the present application will be described in detail below. Generally, the label area 602 has a label substance-antibody conjugated complex, and the testing area 605 is treated with a secondary antibody or an analyte analog for testing. If the secondary antibody is used, the double-antibody sandwich method is used for testing; if the analyte analog is used, the competitive method is used for testing.

### Antibody

The antibody used in the present application may be any antibody capable of specifically binding to an analyte in a sample. The antibody that can be used as a binding agent may be any antibody known to those skilled in the art. The "antibody" may be an immunoglobulin molecule and an antigen binding part of the immunoglobulin molecule, that is, a molecule including an antigen-binding site that specifically binds to an analyte, an analyte analog or a ligand ("immune reaction"). This term also includes derivatives of antibodies in which the binding ability is retained, and also includes any protein including a binding domain that is homologous or largely homologous to a binding domain of an immunoglobulin. These proteins may originate from natural substances, or they may be partially or fully synthesized. An antibody may be monoclonal or polyclonal. An antibody may be a member of any immunoglobulin type, including any human immunoglobulin type: IgG, IgM, IgA, IgD, IgG, and IgE. An "antibody fragment" is a derivative of an antibody or a portion of an antibody that is less than the full length. The antibody fragment can retain at least one significant site of the binding ability of the full-length antibody. Examples of the antibody fragment include an antigen-binding fragment (Fab), an Fab', an F(ab')₂, a single-chain variable fragment (scFv), a variable fragment (Fv), a disulfide-stabilized Fv (dsFv) dimer, and an Fd fragment, but are not limited to the above. The antibody fragment can be generated in any way. For example, the antibody fragment can be generated by enzymolysis or chemical cleavage of a complete antibody, or by recombination from genes that encode partial antibody sequences. In other words, the antibody fragment can be generated by partial or complete recombination. The antibody fragment may be any single-chain antibody fragment. In other words, the antibody fragment may include a plurality of peptide chains linked to each other, for example, by disulfide bonds. The antibody fragment may also be any one of multi-molecular complexes. A functional antibody fragment generally includes at least about 50 amino acids, while more antibody fragments generally include at least about 200 amino acids. Single-chain Fvs (scFvs) are recombinant antibody fragments consisting only of a variable region of light chain (V_{L}) and a variable region of heavy chain (V_{H}) covalently bound to each other in a polypeptide chain. One of V_{L} and V_{H} has an amino terminal region. The length and composition of polypeptide chains are variable, and their length can bridge two variable domains to each other without significantly affecting the arrangement of atoms. Polypeptide chains are generally formed mainly by glycine and serine residue extensions, with some glutamic acid and lysine residues scattered to increase their solubility. The "dimer" refers to a bipolymer of single-stranded Fvs. The monomers of the dimer include peptide chains that are generally shorter than those of most single-chain Fvs, and they show a tendency to form the bipolymer.

The "Fv" fragment is formed by one V_{H} domain and one V_{L} domain non-covalently linked to each other. The term "dsFv" here refers to an Fv including an intermolecular disulfide bond that stabilizes a V_{H}-V_{L} pair. The "F(ab')" fragment is a fragment of an antibody that is essentially identical to the fragment obtained by digesting an immunoglobulin (usually IgG) with pepsin at pH=4.0-4.5. This fragment may also be synthesized by recombination. The "Fab'" fragment is an antibody fragment that is essentially identical to the fragment obtained by reducing the disulfide bond linking the two heavy chains on the F(ab') fragment. The Fab' fragment may also be synthesized by recombination. The "Fab" fragment is an antibody fragment that is essentially identical to the fragment obtained by digesting an immunoglobulin (usually IgG) with papain. The Fab fragment may also be synthesized by recombination. The heavy chain fragment on the Fab fragment is the Fd fragment.

The antibody here may also be any kind of antibody in the analyte, and these antibodies may also be used as the analyte.

### Sample

Samples that can be tested by the test device of the present application include biological liquids (for example, case liquids or clinical samples). Liquid samples or fluid specimens may be derived from solid or semi-solid samples, including excreta, biological tissues, and food samples. Here, a sample and a specimen represent the same meaning and can be used interchangeably. The solid or semi-solid samples can be converted to liquid samples by any appropriate method, such as mixing, mashing, macerating, incubating, dissolving, or digesting the solid samples with enzymolysis in suitable solutions (e.g., water, phosphate solutions, or other buffer solutions). "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine. Food samples include food processed materials, final products, meat, cheese, wine, milk, and drinking water. Plant samples include samples derived from any plant, plant tissue, plant cell culture, and medium. "Environmental samples" include samples derived from the environment (for example, liquid samples from lakes or other bodies of water, sewage samples, soil samples, groundwater, seawater, and waste liquid samples). The environmental samples may further include sewage or other wastewater.

An appropriate testing element according to the present application can be used to test any analyte. Preferably, the present application is used for testing small drug molecules in saliva and urine. In some embodiments, the sample is urine.

### Analyte

In examples where the analyte according to the present application can be used, some semiantigen substances are involved and include drugs (such as drugs of abuse). "Drug of Abuse" (DOA) refers to the use of a drug (typically functions to paralyze the nerves) not directed to a medical purpose. Abuse of these drugs will lead to physical and mental damage, dependency, addiction, and/or death. Examples of abuse of drugs include cocaine; amphetamine (AMP) (e.g., Black Beauty, white amphetamine tablets, dexamphetamine, dexamphetamine tablets, and Beans); methamphetamine (MET) (crank, meth, crystal, and speed); barbiturate (BAR) (such as Valium^{®}, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the present application can also be used for testing drugs belonging to medical use but easy to be taken excessively, such as tricyclic antidepressants (imipramine or analogs) and acetaminophen. These drugs are metabolized into different micromolecular substances after being absorbed by the human body. These micromolecular substances exist in blood, urine, saliva, sweat, and other body fluids or in some body fluids.

For example, the analytes tested according to the present application include but are not limited to creatinine, bilirubin, nitrite, (nonspecific) proteins, hormones (for example, human chorionic gonadotropin, progesterone, and follicle-stimulating hormone), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Campylobacter genus, L. monocytogenes, Vibrio,* or *Bacillus cereus*), and substances related with physiological features in urine samples, such as pH and specific gravity. The chemical analysis of any other clinical urine can be conducted by means of a lateral cross-flow test in combination with the device of the present application.

### Test Device

The test device of the present application can test the presence or absence or quantity of an analyte in a sample by using any technical principle, that is, qualitative or quantitative test. The test device includes a testing element for testing the presence or absence or quantity of an analyte in a sample; in addition to the testing element, the test device may further include a device for accommodating the testing element. The test device of the present application includes a base layer, and the base layer includes a slot for accommodating the testing element and a covering element covering the slot. In this way, the covering element, e.g., a flexible covering layer, can cover or seal the testing element in the slot, such that sample testing can be conducted. At least one end of the slot is sealed while the other end is open. The open end is inserted into a sample to be in contact with the liquid sample, such that the testing element is in contact with the liquid sample for testing. However, if the open end is expected to be rapidly withdrawn from the liquid sample after insertion into the liquid sample, in this case, the testing element may only absorb a very small amount of sample, which is insufficient to be supplied to the testing element to complete the test, and this is the technical problem to be solved by the present application. For example, in such brief contact with the liquid, for example, for 1 second or 2-3 seconds, the volume of the liquid sample absorbed by the testing element is approximately only 1 microliter or 2-3 microliters, however, the liquid volume required for the testing element to complete the test is 10-50 microliters, which is to be solved by the technical solution provided by the present application, i.e., how to enable a device to retain a liquid sample to be supplied to a testing element for continued absorption so as to complete a test, although the device has left the liquid sample.

### Liquid Retaining Structure and Clamping Slot

To provide a new thought for solving the above problems, the test device of the present application includes a base layer 10, the base layer 10 includes one or more slots that are covered by a covering layer 20, such that a testing channel with one sealed end 201 and one open end is formed, and a testing element 60 is located in the channel. A liquid retaining structure is arranged at an opening of the channel. After the open end is inserted into a liquid sample and then needs to be rapidly withdrawn, the retaining structure still retains the liquid sample to continue to be supplied to a test strip for absorption so as to complete a test.

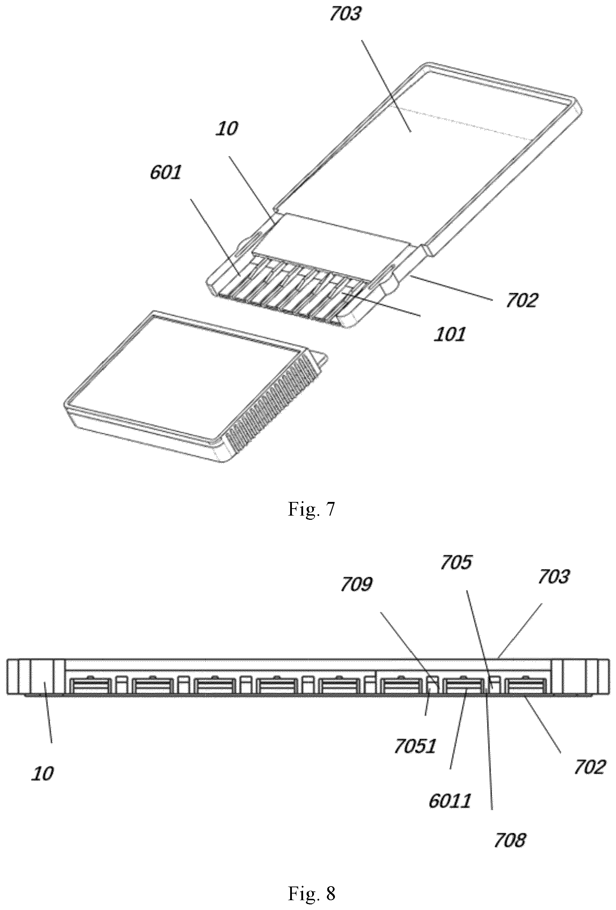

For example, Figs. 6-9 show a test device including multiple slots to form a testing channel. The test device includes a base layer 10. One or more slots are formed in the base layer 10. The base layer 10 has a specific thickness, and slots (also referred to as troughs or multiple ribs having intervals 801) of specific depths can be formed in the base layer. The back of the base layer is covered by a covering layer 702 and the front thereof is covered by a covering layer 703, such that a testing channel with one sealed end 701 and one open end 7011 is formed. Alternatively, a slot is directly formed in the base layer 10. The slot has a specific depth and also a bottom, but the entire slot is open. As shown in Figs. 6-9, a slot 101 is formed in the base layer 10, and the covering layer 703 serves as the bottom of the slot (in this case, the bottom 703 of the base layer is the front of the base layer integrated with the base layer, such as a plastic sheet having a thickness). The width of the bottom of the slot 101 is equivalent to the width for accommodating the testing element, or may be greater than the width of the test strip, for example, a test strip 60; the depth is equivalent to the thickness of the test strip, and the best way is that the depth may be greater than the thickness of the test strip. In this way, when the back is covered by the covering layer 702, a testing channel with one sealed end 701 and one open end 7011 is formed. A section of clamping slot 1011 is formed in an extended section of the testing channel, not covered by the covering layer 703, but exposed (Figs. 8-9). A sample application area 601 of the testing element is arranged in a part of the exposed clamping slot, and an end 6011 of the sample application area aligns with or is slightly shorter than an end 709 of the clamping slot. The exposed slot (open slot 1011) not covered by the covering layer 703 serves as a liquid retaining structure for liquid retaining. The fundamental principle of liquid retaining is to retain a liquid using the dimension of the open slot and the surface tension of the liquid. When one end of the open slot 1011 not covered by the covering layer 703 is inserted into the liquid, the open slot 1011 is immersed in the liquid, meanwhile, a part of the sample application area 601 of the testing element 60 located in the open slot 1011 is in contact with the liquid. After the open slot 1011 is rapidly withdrawn from the liquid, the sample application area 601 can absorb a part of the liquid sample, but is insufficient for completing the test, while the open slot 1011 retains or stores a part of the liquid sample, which enables the testing element to continue absorbing until sufficient liquid sample is acquired so as to complete the entire test.

How to make the open slot 1011 retain the liquid can be solved in multiple different ways. First, the open slot is expected to have the function of preventing the liquid from separating from the slot due to surface tension. For example, one way is that the slot is rough, or any open surface, such as inner side surfaces forming two side walls 705 and 7051 of the slot and a bottom surface 702 (the covering layer 702 serves as the bottom surface of the slot), serves as a rough surface, such that the slot has a higher surface energy that can naturally retain a part of the liquid sample when in contact with the liquid. In addition, a part of the sample application area 601 of the testing element 60 is made of materials such as filter paper or glass fiber to form a rough surface; when these surfaces are inserted into the liquid, they not only absorb the liquid sample but also serve as rough slot surfaces to increase surface energy, thereby retaining more liquid within the open slot.

In some embodiments, the volume of the liquid sample can be retained using the surface tension of the liquid by means of the dimensional configuration of the specific depth or width of the open slot 1011. When considering the depth, the thickness of the sample application area 601 may be subtracted. Different liquid samples have different surface tensions. In some embodiments, if the liquid sample is urine, generally, the surface tension of urine is approximately 50-70 mN/m (millinewtons per meter), of course, the surface tension may change with an increase in temperature and may approach 60-65 mN/m (under conditions close to body temperature, e.g., 37°C). In other words, for the slot according to the present application, the volume of the urine sample that can be retained depends on the surface tension of the open slot determined by the height of a section of two parallel opposing side walls 705 and 7051, or the height of another section of opposing side walls 7021 and 8011, along with the distance (width) between them; alternatively, depends on the depth of the slot determined by the height of the two parallel side walls 705 and 7051, along with the distance between the parallel side walls 705 and 7051 (the volume of urine that can be retained is determined collectively by the width and the depth).

Therefore, in some embodiments, the present application intends to configure the open slot to retain urine within the slot without spillage, thus requiring a specific range of dimensional parameters. This dimensional range enables the realization of the present application. The following theoretical explanation, which serves to provide theoretical support from a scientific and technological perspective, does not impose any limitations on the present application.

First, according to the Laplace formula, surface tension needs to balance the pressure generated by gravity. The formula is ΔP = 2γcosθ / r ≥ ρgh. Here, r represents an open radius. Theoretically, the width of the slot may be more complex as the slot may involve two dimensions, i.e., width and depth. It may be necessary to simplify the problem as considering surface tension effects in the width direction or considering how the geometrical shape of the slot influences liquid retention. The depth of the slot may affect the height of urine (if the depth is determined), but the primary focus lies in the width range. In an embodiment, for example, as shown in Figs. 8-9 of the present application, an open slot 1011 is open, similar to a long and narrow open channel, so liquid retention may depend on the relationship between the surface tension of two side walls 705 and 7051 and the width between them. In this case, the width of the slot may be required as similar to the diameter of a circular opening or considered as an equivalent radius of a rectangular section. In an embodiment, the open slot of the present application is rectangular, so different formulas may be needed, for example, considering the meniscus shape of a liquid. However, for simplification, the Laplace formula may still be used, using width as the characteristic dimension. For example, for a rectangular slot, the effective radius may relate to the width and may take half the width as the equivalent radius.

Assuming the width of the open slot is w and the depth thereof is d, if it is expected to retain the urine height of 1-5 mm in the slot (retaining a liquid sample of a height of 1-5 mm in the sample application area), the depth may at least be equivalent to the height that is at least less than the depth. At this point, the width of the slot needs to be substituted into the above formula, but whether the slot is rectangular or in other shapes needs to be considered. In the present application, if the slot is rectangular, the capillary action between two parallel plates (e.g., two side walls 705 and 7051, 7021 and 8011 of the slot) is considered, for example. At this point, the spacing w between the two plates will affect the rising or retaining height of the liquid. In this case, the capillary height formula is: h = (2γcosθ) / (pg w). If it is expected that h is maintained within 1-5 mm, then the range of w can be derived. For example, h = (2γcosθ) / (pg w), therefore w = (2γcosθ) / (pg h). According to γ=0.06 N/m, θ=0°, ρ=1000 kg/m³, g=9.8 m/s², substituting different h values and calculating w: when h=1 mm=0.001 m, w=(2*0.06*1)/(1000*9.8*0.001)=0.12/(9.8)= approximately 0.01224 m=12.24 mm; when h=5 mm=0.005 m, w=0.12/(1000*9.8*0.005)=0.12/(49)= approximately 0.00245 m=2.45 mm. Therefore, the width range shall be 2.45 mm to 12.24 mm. Thus, if it is expected to retain the urine height of 1-5 mm in the slot, then the width range of the slot is 2.45-12.24 mm; if it is expected to retain the urine height of 5 mm, then the width is 2.45 mm; if the urine depth is a height of 1 mm, then the width may be 12.24 mm.

Although this is a theoretical value, it can at least indicate that by setting different appropriate dimensions for the depth and width of the slot, the present application enables the liquid sample to be retained in the slot through surface tension. For example, it is expected to achieve a liquid level height of 2-3 mm, the suitable dimension can be selected for the width of the slot, e.g., a width of 4-5 mm or 4-8 mm, and the depth may be 2-5 mm. In this way, after the open slot 1011 is inserted into the urine sample and rapidly withdrawn from the urine sample, a specific height of the urine sample can be retained in the open slot 1011, or it is understood that the urine sample can be retained in the open slot 1011. Moreover, when a part of a sample application area is located in the open slot and exposed, it can be in direct contact with the sample and at least can absorb a part of the sample; after the open slot 1011 is rapidly withdrawn from the sample, a specific height of liquid sample can be retained in the open slot 1011, and the retained liquid sample can continue to be supplied to the test strip for absorption and wetting so as to complete the entire test; alternatively, the liquid sample that can be retained in the open slot is sufficient to complete the wetting of the test strip so as to complete the test. After all, liquid flow on the test strip relies on capillary action, which facilitates the absorption and flow of the retained liquid sample on the test strip. Generally, the height of the retained liquid is 1-5 mm; if the width is 2-5 mm, the volume of the liquid sample may be 1 microliter, 25 microliters, or more; any appropriate volume of saturated and wetted liquid of the testing element can be set and is flexibly adjusted based on the liquid volume of the slot retained according to the surface tension of liquid and the liquid absorbed by the testing element itself, and these can be adjusted through simple experiments. Although macroscopically, liquid retention and absorption by the testing element are not easily observable, the time interval between liquid retention in the open slot and rapid absorption by the testing element to complete the test is not very long, and they occur almost simultaneously, we indeed found that the open slot 1011 has the capability to retain a liquid of 10 microliters, 50 microliters, or 100 microliters. When the depth and the width are determined, the longer the length of the open slot, the greater the overall liquid retention capacity.

In this case, the smaller the width, the higher the liquid level that can be retained. Therefore, to retain a height of 1-5 mm, the width shall fall within the range of approximately 2.45 mm to 12.24 mm, or smaller. It can also be considered that when the height of urine required for retention is 5 mm, the width needs to be less than 4.9 mm; when the height required is 1 mm, the width shall be 24.5 mm. At this volume, the length of the open slot also plays a role; the longer the exposed slot, the greater the contact length with the liquid sample, the more the retained liquid; the shorter the exposed slot, the less the retained liquid. These are freely adjustable. Therefore, as shown in Figs. 8-9, although the back covering layer 702 serves as the bottom surface of the slot, the volume of the liquid that can be retained by the slot is determined by the height of parallel side walls 705 and 7051 and the distance between them, along with the length of the exposed slot (not covered by the covering layer 703). Therefore, when the slot is covered by the covering layer 703, a testing channel with one sealed end 701 and one open end 7011 is formed, and the part of the slot not covered by the covering layer 703 serves as a structure for retaining the liquid to be supplied to the testing element for absorption, so the exposed length of the slot is determined by the length of the covering layer 703. It can be understood that any one of the covering layers 703 and 702 can be made of the same material as the base layer 10, for example, the covering layer 703 is made of the same material as the base layer 10, such as plastic or glass, while the covering layer 702 is made of a flexible material; alternatively, the covering layer 702 is made of the same material as the base layer, while the covering layer 703 is made of a flexible material. In addition, the exposed and not covered length and range of the slot are determined depending on the length of the slot covered by the covering layer, for example, both the covering layers 703 and 702 cover a part of the slot while leaving both sides exposed; in this case, by inserting the slot into the liquid, the front and back of the testing element can retain a part of the liquid to be supplied to the test strip for absorption.

Generally, in the manufacturing method, the base layer 10 including the slot is first provided, one side of the base layer is provided with the slot with the length equivalent to the length of the test strip, and the slot is completely exposed; then, the test strip 20 is placed in the slot, with the back side of the test strip facing down (the side with a support structure) and the top side facing up (the side where a testing area or a filter paper can be seen). Then, the slot is covered by the covering layer to form a testing channel with one sealed end and one open end, while reserving a part of the slot unsealed by the covering layer to retain the liquid to be supplied to the testing element for continued absorption. Of course, a part of the sample application area 601 of the test strip is located in the slot not covered by the covering layer. The sample application area 601 and the open slot 1011 are simultaneously inserted into the liquid sample for contact, such that after being rapidly withdrawn from the liquid sample, the open slot 1011 can retain the liquid sample to be supplied to the testing element for absorption. In some embodiments, a label area 602, a testing area 603, and an absorption area 604 downstream of the sample application area 601 are located in a testing channel 7031 from upstream to downstream. In some embodiments, the number of slots may be any number, e.g., 2, 3, 4, 5, 6, 7, 8, 9, 10, 15, or 16, an equal number of testing channels to the slots may be formed, or an equal number of open slots to the slots may be formed, or an equal number of testing elements to the slots may be set, such that an equal number of liquid retaining structures to the slots can be formed, and each open slot can retain the liquid sample to be supplied to the testing element in the slot for absorption.

In some embodiments, the width of the sample application area 601 of the testing element may be the same as the width of the open slot 1011 or slightly smaller than the width of the slot, such that 1-2 mm slits 709 and 708 are formed between the parallel walls of the slot. The slits function like a capillary channel to retain the liquid. When the open slot 1011 is inserted into the liquid sample, the slits having the capillary action can absorb and retain the liquid. After the open slot is rapidly withdrawn from the liquid sample, as the slits having the capillary action retain the liquid, the liquid can continue to be supplied to the test strip for absorption. In some embodiments, the capillary force of the testing element, made for capillary dynamics, is greater than the capillary force of the slits 709 and 708. In this way, the liquid stored in the capillary slits flows onto the testing element via capillary action to be supplied to the testing element for absorption.

In some embodiments, the device is provided with a porous absorbent material, which also serves to absorb and retain the liquid to be supplied to the testing element for continued absorption. In some embodiments, the porous absorbent material 802 is located on the sample application area 601 of the test strip or a part of the sample application area, for direct or indirect contact with the sample application area. In this way, such porous absorbent material can absorb a relatively more liquid sample upon contact. When the sample application area of the test strip is inserted into the liquid sample and rapidly withdrawn from the liquid sample, the porous absorbent material can absorb the liquid to be supplied to the testing element for continued absorption so as to complete the test. In some embodiments, the capillary force of the porous absorbent material is less than the capillary force of the testing element, for example, the larger the pore size of the porous absorbent material, the larger the volume of the liquid absorbed, and the larger pore size can substantially rely on the liquid surface tension for adsorption rather than the capillary force of the porous absorbent material, such as sponges, foam, filter paper, or other large-pore-size materials.

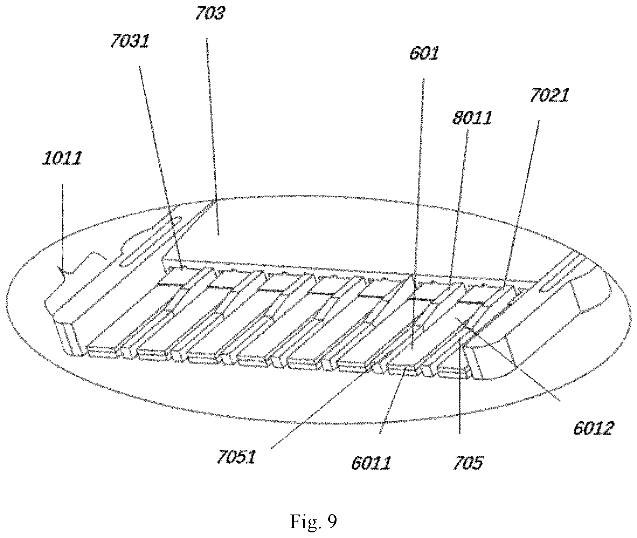

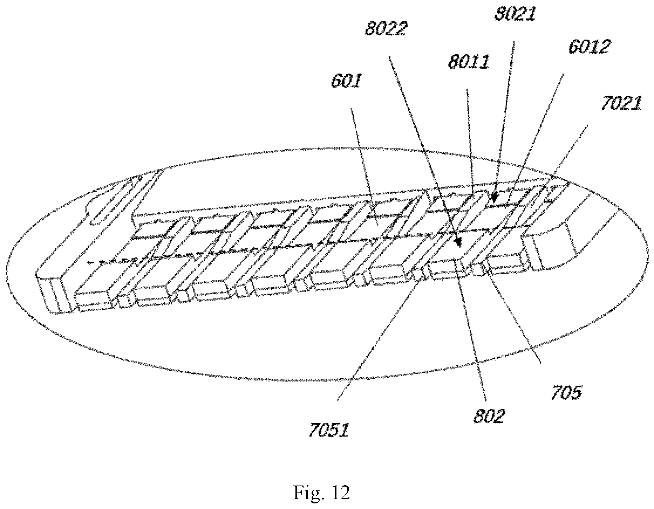

In some embodiments, the porous absorbent material is arranged in the open slot 1011. The absorbent materials 802 are each arranged on a part of the sample application area 601 of the testing element 60 and are also each located in the open slot 1011 (as shown in Figs. 9-12). In some embodiments, the open slot 1011 includes two sections: a first section 8022 and a second section 8021. The first section 8022 is provided with the porous absorbent material 802, the thickness of the additional porous absorbent material and the sample application area is substantially equivalent to the depth of the first section 8022 of the slot; while in the reserved second section 8021 of the slot, the thickness of the part of the sample application area is less than the depth of the second section of the slot (as shown in Fig. 12), which also serves to retain the liquid sample. The side walls 7021 and 8011 in the open slot 1011 of the second section have relatively greater heights, and a part of the sample application area 6012 is also exposed in the second section 8021. When one end of the open slot is inserted into the liquid sample and rapidly withdrawn, the additional porous absorbent material 802 absorbs the liquid that can be supplied to the testing element for absorption; a part of the liquid sample can also be retained in the second section 8021 based on the depth and width of the open slot, and can also be supplied to the test strip for continued absorption, such that the open slot can be rapidly withdrawn from the liquid sample and can continue to absorb the liquid sample so as to complete the test.

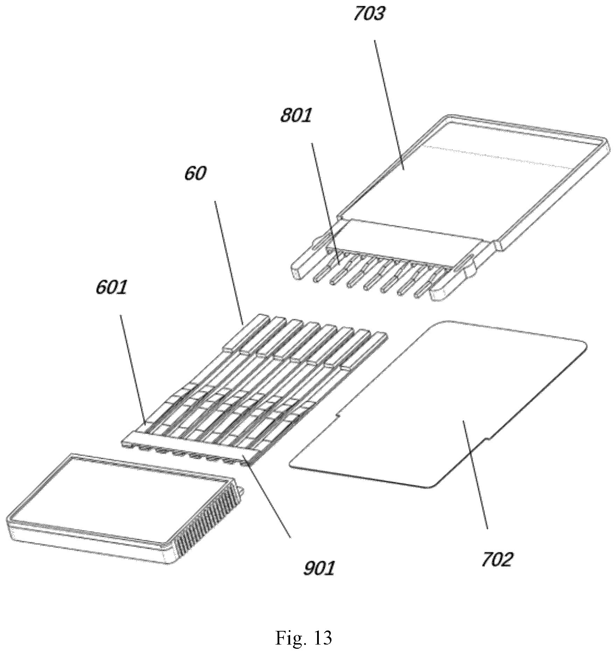

In some embodiments, a porous absorbent material strip can be provided to cover the open slot. For example, as shown in Figs. 13-15, the porous material covers a first section 8022 of an open slot, and the porous absorbent material strip 901 is supported by walls 705 and 7051 of each slot. The absorbent material strip 901 covers the first section 8022 of the slot and may be in direct contact with or not in contact with a sample application area 601 located in the slot. For example, when the covering strip is not in contact with the sample application area 601, a distance, e.g., 1-2 mm or 3-5 mm, exists between the absorbent strip 901 and the sample application area located in the first section 8022. For example, the distance between the parallel walls 705 and 7051 is 5 mm, the height of the walls is 4 mm, the thickness of the sample application area is 1 mm, the covering absorbent strip is on the surfaces of the side walls 705 and 7051, and the distance between the absorbent strip and the sample application area is 3 mm. In this way, when the end with the absorbent strip 901 is inserted into the liquid sample, the liquid is absorbed by the absorbent strip 901, and a part of the liquid also enters a cavity 1080 with a distance of 3 mm between the absorbent strip 901 and the application area 601 and is stored. Meanwhile, the side walls 7021 and 8011 of the second section 8021 are not covered by the porous absorbent strip 901, and a part of the sample application area 6012 is exposed. When the entire open slot 2011 is inserted into the liquid sample, the porous absorbent strip 901 absorbs the liquid, the liquid is stored within the cavity, and the second section of the open slot retains the liquid. After the open slot is rapidly withdrawn from the liquid sample, the liquid sample located in the cavity with a distance of 3 mm between the absorbent strip 901 and the sample application area 601, as well as the liquid absorbed by the absorbent strip 901, and even the liquid retained in the second section of the open slot, can all continue to be supplied to the testing element for absorption so as to complete the test. Of course, the absorbent strip 901 may be in direct contact with the part of the sample application area 601 located in the slot, such that the liquid sample absorbed by the absorbent strip can continue to be supplied to the testing element for absorption so as to complete the test.

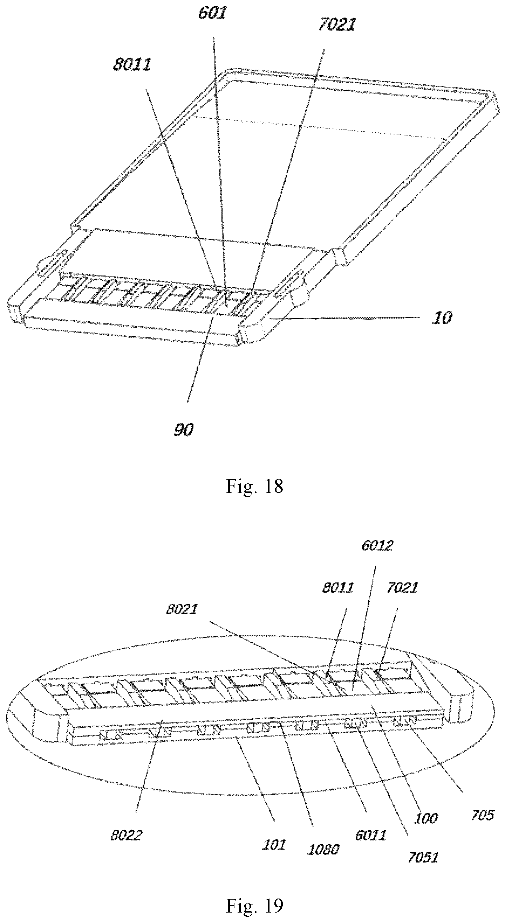

In some other embodiments, the absorbent material 90 wraps a part of the sample application area 601. The wrapping means that the absorbent material forms a rectangular cavity, allowing the sample application area and a part of the open slot to enter and exit from the cavity 90 formed by the absorbent material, allowing a part of the sample application area to be in contact with this absorbent material cavity. In this way, after the end with the absorption cavity 90 is inserted into the liquid sample, the absorption cavity 90 absorbs and retains the liquid sample. After the end with the absorption cavity is rapidly withdrawn from the liquid sample, the liquid sample retained in this absorption cavity 90 can continue to be supplied to the testing element for absorption. In some other embodiments, for example, as shown in Fig. 19, the first area 8022 of the open slot 2011 is covered by a layer of porous absorbent material 100, then the back of the testing element, namely the back of the base layer 10, is covered by a layer of absorbent material 101, and the two layers of materials include a part of the sample application area 601, such that an end 6011 of the testing element is exposed. When the device configured in this manner is inserted into the liquid sample and rapidly withdrawn, the two layers of covering materials 100 and 101 absorb the liquid, the end of the sample application area is in contact with the liquid sample, the exposed part of sample application area 6012 within the second area 8021 is in contact with the liquid sample, and meanwhile, the second area can retain a part of the liquid sample within the open clamping slot. In this way, even if the device is rapidly withdrawn, the entire testing element can be wetted to complete the test.

**In** some embodiments, the amount or volume of liquid absorbed per unit volume of the absorbent material 802, the absorbent strip 901, the cavity, and the porous covering materials 100 and 101 of the present application is greater than or far greater than the amount or volume per unit volume of the sample application area of the testing element. For example, the volume of liquid absorbed per unit volume (1 milliliter or 1 microliter) of the absorbent retention material is 5 milliliters or 10 microliters, while the volume of liquid absorbed per unit volume (milliliter, microliter) of the sample application area may be 1 milliliter or 1 microliter. **In** this way, the liquid sample absorbed in the absorbent material can continue to be supplied to the testing element for absorption so as to complete the test.

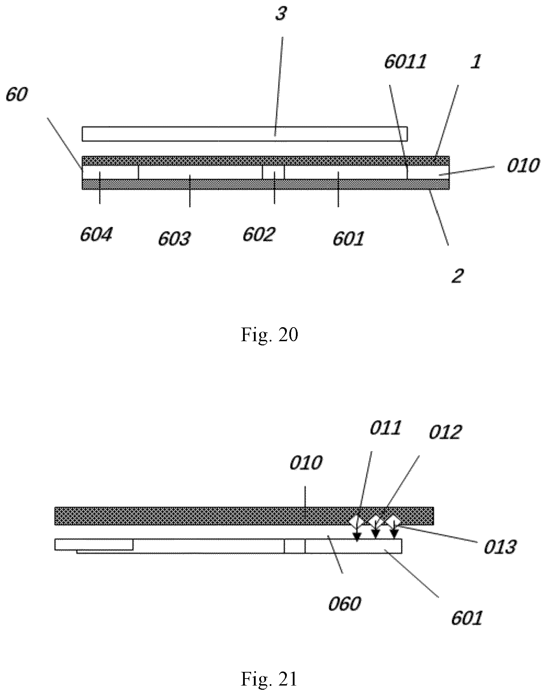

In some embodiments, the device further includes a porous structure for retaining the liquid, for example, as shown in Figs. 20-21, a slot is formed by parallel walls 1 and 2 and a bottom 010. A testing element 60 is arranged in the slot. A sample application area 601, a label area 602, a testing area 603, and an absorbent area 604 of the testing element are arranged in the slot, and then the front side of the testing element 60 covers the bottom 010 of the slot. Similarly, a covering layer 3 covers the slot to form a testing channel with one sealed end and one open end, an end 6011 of the testing element is located in the slot, and a section of the slot is not covered by the covering layer 3 and is exposed to form an open slot. In some embodiments, porous structures 011, 012, and 013 are arranged at the bottom 010 of the slot facing a surface 060 of the sample application area 601 of the test strip. These porous structures can retain the liquid sample. When an end 014 with the porous structures is inserted into the liquid sample, these porous structures can retain the sample. The fundamental principle of retaining the sample is utilizing the action of specific surface tension to retain the liquid in the porous structures 011, 012, and 013. A trace amount of liquid also exists at an inlet and an outlet of the porous structures facing on the surface 060 of the sample application area of the testing element, and between the surface 060 of the sample application area and the porous structures. In this way, through the trace amount of liquid, the liquid in the porous structures 011, 012, and 013 is in connection with the surface 060 of the sample application area on the testing element 60. After the device is rapidly withdrawn from the liquid sample, the test strip may only wet a part of the sample application area and needs to continue absorbing the liquid to wet the label area 602 and the testing area 603. In this way, the liquid in the porous structures 011, 012, and 013 can be absorbed depending on the capillary force of dry areas 602 and 603 downstream of the sample application area 601 so as to complete the entire test. Here, the porous structures may be recessions, holes, cavities, threaded structures, etc.; any structure that can retain the liquid depending on the action of surface tension can serve as part of the present application.

In some embodiments, the present application provides a test device, including one or more testing channels arranged on a base layer. These testing channels have one sealed end and one open end and are all arranged longitudinally. Testing elements are arranged in the channels. The testing element is provided with a sample application area, a label area, a testing area, and an absorption area sequentially from upstream to downstream in the longitudinal direction from the open end to the sealed end. In some embodiments, a flow channel is arranged in the transverse direction of the base layer, the transverse flow channel intersects the longitudinal testing channels and divides the longitudinal testing channels into two parts, i.e., a first area between openings of the longitudinal testing channels and the transverse flow channel, and a second area between the transverse flow channel and the sealed ends of the testing channels. In some embodiments, a part of the sample application area is located in the testing channels within the first area, while the label area and the testing area are located in the testing channels within the second area. When the end with the flow channel is inserted into the liquid sample, the liquid can enter the flow channel from an opening of the flow channel, and the flow channel can function to vent gas. When the flow channel enters the liquid, the liquid can enter the flow channel, and then the liquid can also flow into each testing channel within the first area via the flow channel. That is, the first area is essentially located in the liquid sample, but since one end of the channel in the second area is sealed and the flow channel is open, the liquid essentially does not enter the second area. When the device is rapidly withdrawn from the liquid sample, the liquid retained in the second area (the flow channel and the testing channels) can continue to be supplied to the testing element for absorption. If the liquid is allowed to continue to be absorbed, for example, the liquid retained in the second area continues to be absorbed, then the action of the flow channel is a venting function, facilitating the liquid to be absorbed onto the testing element to continue to complete the test.

How it is implemented will now be explained through specific drawings. For example, as shown in Figs. 1-4, in some embodiments, a base layer 10 is provided with multiple slots 101, all of which are longitudinally distributed. The base layer 10 is provided with a transversely arranged slot 109, and the transverse slot divides the longitudinal slots into two sections 11 and 12. Then, the base layer 10 is covered by a covering layer 20. One end 201 of the covering layer seals the other end of the slots, and the other end 22 covers the other end 14 of the base layer, thereby forming a testing channel with one open end and one sealed end or multiple parallel testing channels. The length of the covering layer 20 or the length between ends 201 and 22 is the same as or slightly greater than the length of the longitudinal slots. The transverse width 23-24 of the covering layer is the same as or slightly greater than the width of the base layer, such that the entire slots can be covered to form a testing channel. Meanwhile, the covering layer also forms a flow channel passing through the entire slot 109, and the flow channel has a flow channel 108 with two open ends 105 and 104. It may also be considered that the flow channel 108 divides the multiple testing channels into two areas. Multiple parts of testing channels are distributed on the first area 11 and are in communication with the flow channel, or the flow channel is in parallel connection with multiple parts of testing channels 102, 105, 106, and 107. In this specific embodiment, the flow channel is in parallel connection with 8 parts of testing channels, which are located within the first area 11, while the other parts of the testing channels 12 are located within the second area separated by the flow channel 108; the parts of the testing channels within the first area and the testing channels within the second area 12 are located in an integrated clamping slot and are divided into two segments by the transverse flow channel 108. A testing element 60 is arranged in the testing channel, wherein a part of a sample receiving area 601 is located in a part of the testing channel on the first area, and a label area 602 is located on a recessed area 402, which is located downstream of the flow channel 108, while a testing area 608 downstream of the label area 602 is located in the testing channel within the second area. Meanwhile, an end 6011 of the testing element aligns with an opening 1021 of the testing channel within the first area 11. In the specific embodiment shown in this view, 8 testing elements are respectively arranged in the 8 testing channels. During testing, one end of the flow channel 108 is inserted into the liquid sample, the liquid sample can enter each testing channel within the first area of the test device from two directions, and the liquid can enter the flow channel from two openings 105 and 104 of the flow channel 108, and then flow into the parts of the testing channels on the first area through the flow channel 108. Gas is vented through the openings 1021 of the parts of the testing channels, facilitating rapid inflow. If the liquid sample enters each testing channel on the first area from the openings 1021, gas can be vented through the flow channel 108, facilitating rapid entry of the liquid into the parts of the testing channels on the first area 11. Because the parts of the testing channels on the first area 11 have liquid surface tension, the liquid can be retained in the parts of the testing channels, for example, the parts of the testing channels are 4-6 mm wide and 2-4 mm high, allowing a part of the liquid sample to be retained within them. At this point, the parts of the testing channels within the second area 12 including recessed areas are sealed, preventing the liquid from entering recesses 402, 405, and 401. However, the sample application areas in the parts of the testing channels located on the first area 11 can also absorb the liquid. After the end with the flow channel 108 is inserted into the liquid sample for a brief time and then rapidly withdrawn, the liquid retained in a part of the first area 11 can continue to be supplied to the testing elements for absorption so as to complete the test. The purpose of arranging the recessed areas downstream of the flow channel 108 is to prevent the liquid that enters the flow channel from entering the testing channels of the second area 12. If an excess liquid is present, it will flow into the recessed areas for storage, preventing the excessive liquid from entering the testing channels of the second area 12.

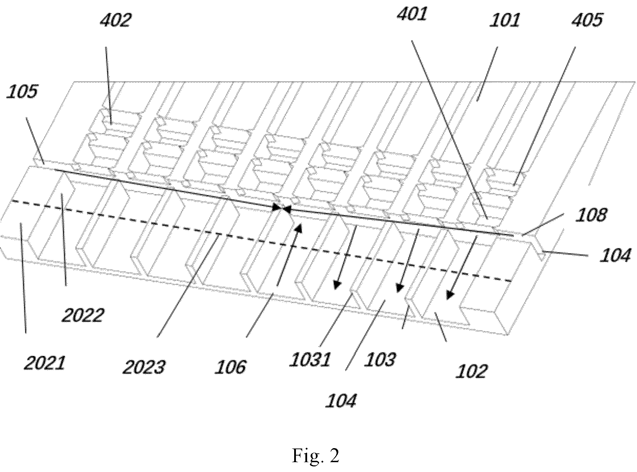

In some embodiments, the covering layer 20 is slightly shorter than the entire slots forming the testing channels. As shown in Fig. 2, the end 22 of the covering layer only covers the flow channel 108 or extends beyond it, but merely covers parts of the slots within a part of the first area, for example, the dotted line 2023 refers to where the end of covering layer 22 aligns. Thus, downstream of the dotted line 2023 lie parts of testing channels, covered by the covering layer, while upstream of the dotted line lie the open slots. In practice, the slots within the first area are divided into two parts: one part 2022 covered by the covering layer to form parts of the testing channels, and another part 2021 not covered by the covering layer to be in an open form to form open slots. Parts of the sample application areas 601 are arranged in the testing channel/open slots of the entire first area, and located in both the part of the testing channels of the first part 2022 and the open slots not covered by the covering layer. When the end with the flow channel 108 is inserted into the liquid, the liquid can enter the flow channel through two open ends 104 and 105, the liquid entering the flow channel then enters the part of the testing channels in parallel, gas within the part of the testing channels in parallel is vented, and the liquid remains in the part of the testing channels, and the open slots can retain a part of the liquid due to liquid surface tension. After insertion into the liquid and rapid withdrawal, the liquid retained in the open slots, the liquid sample retained in the flow channel 108, and a part of the liquid sample retained within the first area 11 upstream of the flow channel 108 can all continue to be supplied to the testing elements 60 for absorption so as to complete the test. The design of the present application is on the basis that the liquid enters the transversely arranged flow channel and is distributed to the part of the testing channels in parallel connection, such that the liquid can wet the testing elements and can also be retained within the testing channels to be supplied to the testing elements for continued absorption so as to complete the test.

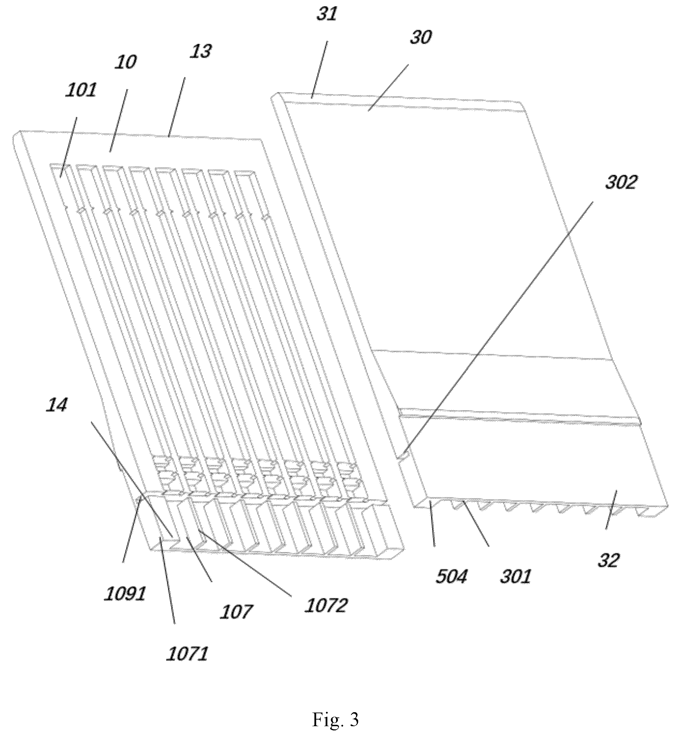

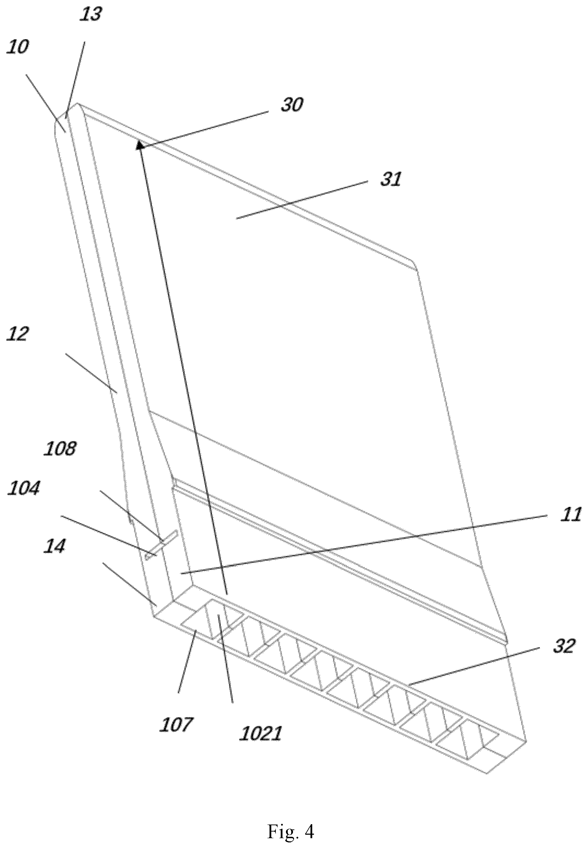

In some embodiments, the test device includes a base layer 10 and a covering layer 30. The base layer 10 and the covering layer 30 are provided with side walls 1071, 1072, 301, and 504, and flow slots 1091 and 302, respectively. When the base layer 10 is covered by the covering layer, the flow slots are combined into a flow channel 108, while slots are combined into testing channels 107 (as shown in Fig. 4). One end 13 of the testing channel is sealed, and the other end is an opening 1021. Meanwhile, the flow channel 108 traverses all the testing channels and divides them into two parts: one part is a first area 11 from the openings to the flow channel, and the other part is a second area 12 from the flow channel 108 to the sealed other end. The testing channels within the first area and the testing channels within the second area are separated by the flow channel 108 and are connected together. Test strips are arranged in the testing channels within the first area and the second area in the following manner, for example, as shown in Fig. 3: in the slots of the base layer 10, one testing element is arranged in each slot, meanwhile, a part of the sample application areas 601 are located in the slots within the first area, and after the covering layer 30 is combined with the base layer 10, the part of the sample application areas 601 are located in the testing channel within the first area 11. In some embodiments, the part of the testing areas may also be located in the flow channel or traverse the flow channel 108; the flow channel may be circular, rectangular, or in any other shape. The flow channel has two openings 104 and 105 at both ends of the channel. The testing channels 107 within the first area are formed by the combination of the side walls 504 and 301 of the covering layer 30 and the side walls 1071 and 1072 of the base layer 10. In this way, the multiple testing channels within the first area 11 are independent of each other but are all in communication with the flow channel 108. Multiple testing channels are also distributed within the second area 12 downstream of the flow channel 108, one end of the testing channels is sealed, and the other end is connected to the flow channel 108. When the end with the flow channel 108 is inserted into the liquid sample, in practice, the liquid can enter the testing channels within the first area through two inlets. The first is to enter the flow channel 108 through the openings 104 and 105 at both ends of the flow channel. The liquid entering the flow channel 108 enters each testing channel within the first area respectively. The part of the sample application areas located in the flow channel or located in the testing channel within the first area will be in contact with the liquid. If an excess liquid is present, it will be retained in the testing channels within the first area. After the inserted end is rapidly withdrawn from the liquid sample, the liquid retained in the testing channels within the first area can continue to be supplied to the testing elements for absorption so as to complete the test. In this embodiment, the opening 1021 of the testing channel within the first area may be set to any size, for example, it may be set to a size that satisfies the liquid surface tension without spillage, such as the size of the opening 1022 of the testing channel within the first area limiting the liquid retained in the testing channel from flowing out, e.g., a 1-2 mm or 2-4 mm opening. When the liquid enters the flow channel through the openings 104 and 105 of the flow channel and then enters the respective testing channels on the first area 11, the opening 1021 mainly serves for venting. After the liquid fills the testing channels within the first area, the liquid will not flow out from opening 1022, such that the liquid can be better retained in the testing channels within the first area; at this time, the testing channel in the first area resembles a cavity, the opening 1021 at the bottom of the cavity serves as a venting hole, and the venting hole has a certain size that prevents the liquid from flowing out through the opening 1022. In some other embodiments, the size of the flow channel can be set so that when the end with the flow channel is inserted into the liquid sample, the liquid can enter the testing channels with the first area from the end with the opening 1021. To facilitate liquid entry, the flow channel mainly serves for venting, and the liquid does not easily flow out from the flow channel 108. Pat of the liquid entering the testing channels with the first area wets the sample application areas of the test strip, and the other part is retained in the testing channels within the first area based on the liquid surface tension. After the test device is inserted into the liquid sample and rapidly withdrawn, the liquid sample retained by the testing channels within the first area can continue to be supplied to the testing elements so as to complete the test.

The "continue" here means that when the test strip is in contact with the liquid for a short time, the absorbed liquid is insufficient for completing the test, or it is insufficient for wetting the reagent area and the testing area downstream of the test sample application area, and the liquid retaining structure also contains the liquid, which can continue to be supplied to the test strip for absorption, thus ensuring that the liquid can wet the reagent area and the testing area. Of course, preferably, the liquid can also wet the absorbent area. In this way, the test strip in the device can be in contact with the liquid sample quickly, for example, for 1 second, 2 seconds, 3 seconds, 4 seconds, 5 seconds, 10 seconds, or 20 seconds, thereby improving the testing efficiency and ensuring valid test results.

The invention shown and described herein can be realized without any element or limitation specifically disclosed herein. Terms and expressions used herein are used as terms of the description rather than limitation, and it is not intended to exclude any equivalents of the features shown and described or parts thereof in the use of these terms and expressions, and it should be recognized that various modifications are feasible within the scope of the present application. Therefore, it should be understood that although the present application has been specifically disclosed through various embodiments and optional features, the modifications and variations of the concepts described herein can be adopted by those skilled in the art, and these modifications and variations are considered to fall within the scope of the present application as defined in the appended claims. The contents of articles, patents, patent applications, and all other documents and electronically available information described or recorded herein are incorporated by reference in their entirety to some extent, just as each individual publication is specifically and individually indicated for reference. The applicant reserves the right to incorporate any and all materials and information from any such articles, patents, patent applications, or other documents into this application.

## Claims

1. A test device for testing an analyte in a liquid sample, comprising:
a base layer, provided with a slot for accommodating a testing element; and
a covering layer, covering a part of the slot to form a testing channel with one sealed end and one open end, wherein the other part of the slot not covered by the covering layer forms an open slot as a liquid retaining structure, and after one end with the open slot is inserted into the liquid sample and then rapidly withdrawn from the liquid sample, the open slot retains a part of the liquid sample based on liquid surface tension to be supplied to a test strip in the slot for continued absorption so as to complete a test.

2. The test device according to claim 1, wherein the testing element is arranged in the testing channel, and a part of a liquid sample application area of the testing element is located in the open slot.

3. The test device according to any one of claims 1-2, wherein the testing element further comprises a label area and a testing area located downstream of the liquid sample application area, the testing area and the label area are located in a sealed channel, and the part of the liquid sample application area is located in the open slot.

4. The test device according to any one of claims 1-3, wherein a depth of the open slot is greater than a thickness of the liquid sample application area.

5. The test device according to claim 4, wherein the open slot has a depth of 2-8 mm or/and a width of 2-8 mm.

6. The test device according to any one of claims 4-5, wherein the open slot has a depth of 2-6 mm.

7. The test device according to any one of claims 1-6, wherein the base layer comprises a back surface and a front surface, the slot is located in the back surface or the front surface of the base layer, and the covering layer covers the part of the slot of the front surface or the back surface of the base layer.

8. The test device according to any one of claims 1-7, wherein the liquid retaining structure comprises the open slot, and a width of the open slot is greater than a width of a sample application area of the test strip, such that a gap for retaining the liquid sample is formed through the slot and the sample application area.

9. The test device according to any one of claims 1-8, wherein the slot is formed by two side walls arranged oppositely and a bottom, a part of a sample application area is located on the bottom, and fences have a width greater than a width of the sample application area to form a gap, such that the gap is capable of retaining the part of the liquid sample therein through surface tension to be supplied to the sample application area for absorption.

10. The test device according to any one of claims 1-9, further comprising an absorbent porous material, wherein the absorbent porous material covers a sample application area and is configured to absorb and retain the liquid sample, the liquid sample is absorbed by the sample application area, and the absorbent porous material is located in the open slot.

11. The test device according to any one of claims 1-10, further comprising an absorbent porous material strip covering multiple open slots, wherein liquid storage spaces exist between the absorbent porous material strip and application areas of testing elements in the open slots, and when one end with the absorbent porous material strip is inserted into the liquid sample, the liquid sample is absorbed by the absorbent porous material strip and retained by the liquid storage spaces to be supplied to the testing elements for continued absorption so as to complete the test.

12. A test device for testing an analyte in a liquid sample, comprising:
a base layer, provided with multiple longitudinally arranged slots for accommodating a testing element;
a covering layer, covering the multiple slots to form multiple testing channels with one sealed end and one open end, wherein the testing element is arranged in each testing channel; and
a flow channel, traversing the multiple testing channels and provided with openings, wherein the flow channel divides the multiple testing channels into a first area and a second area, the first area is an area between openings of the testing channels to the flow channel, and the second area is an area between the flow channel and sealed ends; a part of a sample application area of the testing element is located in the testing channel and the flow channel within the first area; and the flow channel is in fluid communication with the multiple testing channels within the first area.

13. The test device according to claim 12, wherein a label area and a testing area of the testing element are located in the testing channel within the second area.

14. The test device according to any one of claims 12-13, wherein when one end with the flow channel is inserted into the liquid sample and then rapidly withdrawn, the liquid sample enters the flow channel via the openings of the flow channel and then enters the testing channels within the first area, wherein a part of the liquid sample entering the testing channels within the first area is absorbed by the sample application areas located in the testing channels within the first area, and the other part of the liquid sample is retained in the testing channels within the first area to be supplied to the sample application areas for continued absorption so as to complete a test.

15. The test device according to any one of claims 12-14, wherein the openings of the testing channels within the first area have a size that is capable of retaining the liquid sample in the testing channels without spillage.

16. The test device according to any one of claims 12-15, wherein when one end with the flow channel is inserted into the liquid sample and then rapidly withdrawn, the liquid sample enters the testing channels via the openings of the testing channels within the first area and then enters the flow channel, a part of the liquid sample is absorbed by the sample application areas located in the testing channels within the first area, and the other part of the liquid sample is retained in the testing channels within the first area to be supplied to the testing elements for continued absorption so as to complete a test.

17. The test device according to any one of claims 12-16, wherein when the liquid sample enters the testing channels within the first area, the flow channel serves as a venting channel to allow the liquid sample to faster enter the testing channels within the first area.
